# EUROPÄISCHE PATENTSCHRIFT

(11) **EP 2 790 788 B1**
(45) Veröffentlichungstag und Bekanntmachung des Hinweises auf die Patenterteilung: **01.01.2020**
(21) Anmeldenummer: 12783609.6
(22) Anmeldetag: 09.11.2012
(51) Int. Cl.: A61K 8/81, A61Q 5/06

(54) **MITTEL ZUR TEMPORÄREN VERFORMUNG KERATINISCHER FASERN UND VERFAHREN ZUR TEMPORÄREN HAARVERFORMUNG**
MEDIUM FOR THE TEMPORARY DEFORMATION OF KERATIN FIBRES AND METHOD FOR TEMPORARY HAIR DEFORMATION
AGENT POUR LA MISE EN FORME TEMPORAIRE DE FIBRES KÉRATINIQUES ET PROCÉDÉ POUR LA MISE EN FORME TEMPORAIRE DE CHEVEUX

(30) Priorität: 16.12.2011 DE 102011088845
(43) Veröffentlichungstag der Anmeldung: 22.10.2014
(73) Patentinhaber: Henkel AG & Co. KGaA, 40589 Düsseldorf (DE)
(72) Erfinder: METTEN, Diane, 22393 Hamburg (DE); RICHTERS, Bernd, 21129 Hamburg (DE)
(86) Internationale Anmeldenummer: PCT/EP2012/072218
(87) Internationale Veröffentlichungsnummer: WO 2013/087308

(56) Entgegenhaltungen:
- WO-A1-99/13841
- WO-A1-2012/075274
- "ISP launches Styleze XT3, latest polymer technology to protect hair from heat", INTERNET CITATION, 1. April 2011 (2011-04-01), XP002695780, Gefunden im Internet: URL:http://www.specialchem4cosmetics.com/s ervices/news.aspx?id=6408
- "Styleze XT3 polymer", Ashland.com , 2012, XP002698833, Gefunden im Internet: URL:http://www.innovadex.com/documents/115 6114.pdf?bs=4989&b=200302&st=20 [gefunden am 2013-06]

## Beschreibung

Die vorliegende Anmeldung beschreibt kosmetische Mittel auf Grundlage einer spezifischen Polymerkombination, die Verwendung dieser kosmetischen Mittel zur temporären Verformung keratinischer Fasern sowie kosmetische Verfahren unter Einsatz dieser Mittel.

Der Einsatz von Polymeren in verschiedensten kosmetischen Mitteln ist weit verbreitet. Sie finden sich in Mitteln zur Behandlung der Haut ebenso wie in Mitteln zur Behandlung der Haare, in Mitteln, die unmittelbar nach der Anwendung wieder ab- bzw. ausgewaschen werden, so genannten Rinseoff Produkten, genauso wie in Mitteln, die auf Haut oder Haar verbleiben, so genannten Leave-on Mitteln. Dabei werden die Polymere aus unterschiedlichsten Gründen eingesetzt und jeweils bestimmte Eigenschaften der Polymere ausgenutzt. In Mitteln zur Hautbehandlung, in Shampoos, Haarspülungen und Haarkuren stehen oftmals die verdickenden oder pflegenden Eigenschaften der Polymere im Vordergrund. In Mitteln zur temporären Verformung keratinischer Fasern, im Folgenden auch Stylingmittel genannt, sind neben diesen Eigenschaften vor allem filmbildende und/oder festigende Wirkungen gefragt. Oftmals dienen Polymere auch als Hilfsmittel um die Abscheidung und Fixierung anderer Wirk- und Inhaltsstoffe auf der Haut oder dem Haar zu verbessern oder erst möglich zu machen. So lassen sich beispielsweise durch Zusatz geeigneter Polymere zu Haarfärbemitteln die Reibechtheiten und die Beständigkeit der Färbung erhöhen.

In der Regel enthalten kosmetische Mittel einzelne Polymere, die speziell darauf zugeschnitten sind, einen ganz bestimmten Effekt zu erzielen. Sollen verschiedene Effekte erzielt werden, ist die Zugabe mehrerer Polymere erforderlich. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.

Es besteht daher ständig Bedarf an Polymeren oder geeigneten Kombinationen weniger Polymere, die möglichst viele der gewünschten Eigenschaften gleichzeitig aufweisen.

Beispielsweise ist es im Falle der Stylingmittel notwendig, dass die eingesetzten Polymeren dem behandelten Haar einen möglichst starken Halt geben. Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein. Handelt es sich bei dem Stylingmittel um ein Gel oder eine Paste, sollen die Polymere zudem verdickende Eigenschaften besitzen.

Die Aufgabe der vorliegenden Erfindung war es daher, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen.

Diese Aufgaben wurden durch eine spezielle Polymerkombination gelöst. Ein erster Gegenstand der Erfindung ist ein kosmetisches Mittel, enthaltend in einem kosmetisch akzeptablen Träger
a) mindestens ein Copolymer A mit mindestens einer Struktureinheit (A1), mindestens
   einer Struktureinheit (A2), mindestens eine Struktureinheit (A3) und mindestens einer Struktureinheit (A4), 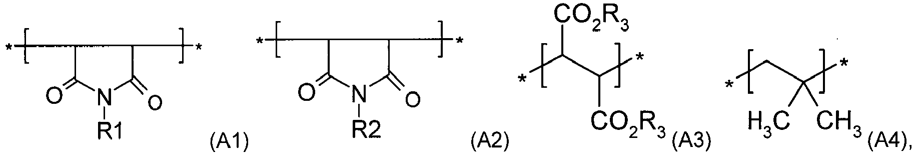 wobei
   - R1 für einen gegebenenfalls heterofunktionaliserten Alkylrest steht;
   - R2 für einen von R1 verschiedenen, gegebenenfalls heterofunktionalisierten Alkylrest steht;
   - R3 voneinander unabhängig für einen von R1 und R2 verschiedenen gegebenenfalls heterofunktionalisierten Alkylrest steht, wobei der Gewichtsanteil des Copolymers A am Gesamtgewicht des Mittels 0,05 bis 10 Gew.-% beträgt
b) mindestens ein von Copolymer A verschiedenes Copolymer B, welches vollständig aus einer Struktureinheit (B1) 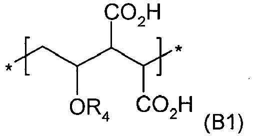 besteht, wobei R4 für einen gegebenenfalls heterofunktionaliserten Alkylrest steht und der Gewichtsanteil des Copolymers B am Gesamtgewicht des Mittels 0,05 bis 10 Gew.-% beträgt; und
c) mindestens ein von Copolymer A und Copolymer B verschiedenes Copolmyer C, gebildet aus
   - mindestens zwei Monomeren C1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
   - Methacryloylethylaminoxid als Monomer C2,
wobei der Gewichtsanteil des Copolymers C am Gesamtgewicht des Mittels 0,1 bis 8,0 beträgt.

Die erfindungsgemäßen Mittel enthalten die Wirkstoffe in einem kosmetischen Träger. Dieser kosmetische Träger ist wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Bevorzugte kosmetische Mittel enthalten, bezogen auf sein Gesamtgewicht, 40 bis 99 Gew.-%, vorzugsweise 50 bis 98 Gew.-%, besonders bevorzugt 60 bis 95 Gew.-% und insbesondere 70 bis 90 Gew.-% Wasser. Besonders bevorzugte kosmetische Mittel umfassen 60 bis 99 Gew.-%, vorzugsweise 75 bis 98 Gew.-% und insbesondere 85 bis 95 Gew.-% Wasser und 0,1 bis 20 Gew.-%, vorzugsweise 0,5 bis 10 Gew.-% und insbesondere 1,0 bis 7,0 Gew.-% C₁-C₄-Alkohol, vorzugsweise Ethanol.

Die erfindungsgemäßen Mittel enthaltend als ersten wesentlichen Bestandteil ein Copolymer A, umfassend die Struktureinheiten (A1), (A2), (A3) und (A4). Für die technische Wirkung der erfindungsgemäßen Mittel hat es sich als vorteilhaft erwiesen, wenn das Copolymer A zu mindestens 70 Gew.-%, vorzugsweise zu mindestens 80 Gew.-%, bevorzugt zu mindestens 90 Gew.-% und insbesondere zu mindestens 95 Gew.-% aus den Struktureinheiten (A1), (A2), (A3) und (A4) besteht. Weitere bevorzugte Copolymere A bestehen vollständig aus den Struktureinheiten (A1), (A2), (A3) und (A4).

In einer bevorzugten Ausführungsform steht der Rest R1 in der Struktureinheit (A1) für einen Etherrest, vorzugsweise für einen polyalkoxylierten Rest. Besonders bevorzugt werden Struktureinheiten (A1), in denen R1 in Formel (A1) für einen Rest -CH(CH₃)CH₂-(OCH(CH₃)CH₂)ₓ(O[CH₂]₂)_{y}OCH₃ steht, in dem x und y unabhängig voneinander einen Wert zwischen 1 und 100 aufweisen.

Der Rest R2 in der Struktureinheit (A2) steht vorzugsweise für einen aminogruppenhaltigen Rest, vorzugsweise für einen Rest mit einem tertiären Amin. Besonders bevorzugt werden Struktureinheiten (A2), in denen R2 in Formel (A2) für einen Rest -(CH₂)₃-N(CH₃)₂ steht.

In der Struktureinheit (A3) wird als Rest R3 vorzugsweise mindestens ein Alkylrest, bevorzugt ein C1 bis C4 Alkylrest eingesetzt. Besonders bevorzugt werden Struktureinheiten (A3), in denen R3 für -CH₂CH₃ oder -CH₂CH₂CH₃, vorzugsweise für -CH₂CH₃ und ein Rest R3 in Formel (A3) für H steht.

Einige bevorzugte Copolymere A sind nachfolgend aufgeführt. Diese Copolymere A bestehen zu mindestens 70 Gew.-%, vorzugsweise zu mindestens 80 Gew.-%, bevorzugt zu mindestens 90 Gew.-% und insbesondere zu mindestens 95 Gew.-%, ganz besonders bevorzugt vollständig aus den Struktureinheiten (A1), (A2), (A3) und (A4):
A-I) Copolymer A mit mindestens einer Struktureinheit (A1), mindestens einer Struktureinheit (A2), mindestens eine Struktureinheit (A3) und mindestens einer Struktureinheit (A4), 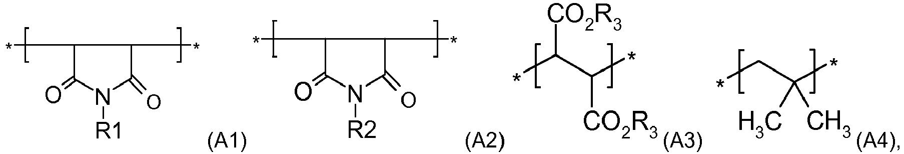 wobei
   - R1 in Formel (A1) für einen Rest -CH(CH₃)CH₂-(OCH(CH₃)CH₂)ₓ(O[CH₂]₂)_{y}OCH₃ steht, in dem x und y unabhängig voneinander einen Wert zwischen 1 und 100 aufweisen;
   - R2 in Formel (A2) für einen Rest -(CH₂)₃-N(CH₃)₂ steht;
   - R3 voneinander unabhängig für einen von R1 und R2 verschiedenen gegebenenfalls heterofunktionalisierten Alkylrest steht.
A-II) Copolymer A mit mindestens einer Struktureinheit (A1), mindestens einer Struktureinheit (A2), mindestens eine Struktureinheit (A3) und mindestens einer Struktureinheit (A4), 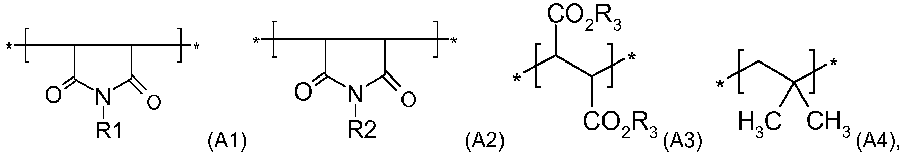 wobei
   - R1 in Formel (A1) für einen Rest -CH(CH₃)CH₂-(OCH(CH₃)CH₂)ₓ(O[CH₂]₂)_{y}OCH₃ steht, in dem x und y unabhängig voneinander einen Wert zwischen 1 und 100 aufweisen;
   - R2 für einen von R1 verschiedenen, gegebenenfalls heterofunktionalisierten Alkylrest steht;
   - ein Rest R3 in der Formel (A3) für -CH₂CH₃ oder -CH₂CH₂CH₃, vorzugsweise für -CH₂CH₃ und ein Rest R3 in Formel (A3) für H steht.
A-III) Copolymer A mit mindestens einer Struktureinheit (A1), mindestens einer Struktureinheit (A2), mindestens eine Struktureinheit (A3) und mindestens einer Struktureinheit (A4), 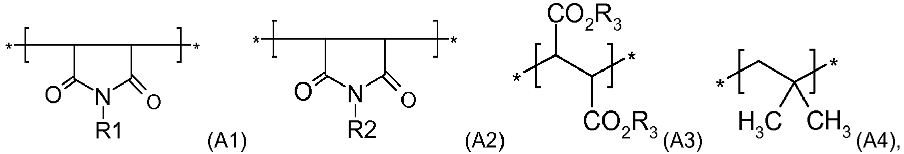 wobei
   - R1 für einen gegebenenfalls heterofunktionaliserten Alkylrest steht;
   - R2 in Formel (A2) für einen Rest -(CH₂)₃-N(CH₃)₂ steht;
   - ein Rest R3 in der Formel (A3) für -CH₂CH₃ oder -CH₂CH₂CH₃, vorzugsweise für -CH₂CH₃ und ein Rest R3 in Formel (A3) für H steht.

Ganz besonders bevorzugte erfindungsgemäße kosmetische Mittel sind dadurch gekennzeichnet, dass
- der Rest R1 in Formel (A1) für einen Rest -CH(CH₃)CH₂-(OCH(CH₃)CH₂)ₓ(O[CH₂]₂)_{y}OCH₃ steht, in dem x und y unabhängig voneinander einen Wert zwischen 1 und 100 aufweisen;
- der Rest R2 in Formel (A2) für einen Rest -(CH₂)₃-N(CH₃)₂ steht;
- ein Rest R3 für -CH₂CH₃ und ein Rest R3 in Formel (A3) für H steht.

Der Gewichtsanteil des Copolymers A am Gesamtgewicht erfindungsgemäßer kosmetischer Mittel beträgt 0,05 bis 10 Gew.-%, vorzugsweise 0,1 bis 7,0 Gew.-% und insbesondere 0,2 bis 5,0 Gew.-%.

Als zweiten wesentlichen Bestandteil enthalten die erfindungsgemäßen Mittel ein Copolymer B, bestehend vollständig aus der Struktureinheit (B1).

In einer bevorzugten Ausführungsform steht der Rest R4 in der Struktureinheit (B1) für einen Alkylrest, bevorzugt einen C1 bis C4 Alkylrest. Besonders bevorzugt wird die Struktureinheit (B1), in welcher der Rest R4 für -CH₃ steht.

Der Gewichtsanteil des Copolymers B am Gesamtgewicht erfindungsgemäßer kosmetischer Mittels beträgt 0,05 bis 10 Gew.-%, vorzugsweise 0,1 bis 7,0 Gew.-% und insbesondere 0,2 bis 5,0 Gew.-%.

Die Copoylmere A und B können in das erfindungsgemäße Mittel in Reinform eingearbeitet werden. Für die Verarbeitung und die kosmetischen Eigenschaften der erfindungsgemäßen kosmetischen Mittel hat es sich jedoch als vorteilhaft erwiesen, die Copolymere A und B in vorkonfektionierter Form, also in Kombination mit weiteren Wirk- oder Hilfsstoffen, einzusetzen. Bevorzugt werden dabei insbesondere Gemische aus den Copolymeren A und B eingesetzt. Das Gewichtsverhältnis des Copolymers A zum Copolymer B in bevorzugt eingesetzten Polymergemische beträgt 20:1 bis 1:20, vorzugsweise 10:1 bis 1:10, insbesondere 8:1 bis 1:8 und ganz besonders bevorzugt 5:1 bis 1:5. Folgerichtig sind daher auch erfindungsgemäße kosmetische Mittel bevorzugt, in denen das Gewichtsverhältnis des Copolymers A zum Copolymer B 20:1 bis 1:20, vorzugsweise 10:1 bis 1:10, insbesondere 8:1 bis 1:8 und ganz besonders bevorzugt 5:1 bis 1:5 beträgt. Ganz besonders bevorzugt ist der Einsatz der Copoylmere A und B in einem Gewichtsverhältnis von 2:1 bis 1:4, vorzugsweise von 1:1 bis 1:3. Vorzugsweise wird das Copolymer B im Überschuss eingesetzt.

Die zuvor beschriebenen Copolymere A und B oder deren Gemische werden vorzugsweise mit zusätzlichen Hilfsstoffen kombiniert. Besonders bevorzugt ist der Einsatz von Alkoholen. Eine bevorzugte Klasse von Alkoholen sind die Diole, insbesondere die 1,2 Diole. Mit besonderem Vorzug wird 1,2 Octandiol eingesetzt. Insbesondere das 1,2 Octandiol erleichtert nicht nur die weitere Verarbeitung der Copolymere A und B, bzw. ihrer Gemische, sondern verstärkt zusätzlich noch deren vorteilhafte technische Wirkung, insbesondere deren pflegende Wirkung.

Die vorgenannten Diole können gemeinsam mit den Copolymeren A und B, bzw. deren Gemisch, oder separat von den Copolymeren A und B in die erfindungsgemäßen kosmetischen Mittel eingearbeitet werden. Vor diesem Hintergrund sind erfindungsgemäße kosmetische Mittel bevorzugt, die weiterhin mindestens ein 1,2 Diol, vorzugsweise 1,2 Octandiol enthalten.

Ein erfindungsgemäß bevorzugtes Polymergemisch auf Grundlage der Copolymere A und B, wird unter der Bezeichnung Styleze® XT3 (INCI: Water (and) Polyimide-1 (and) PVM/MA Copoylmer (and) Caprylyl Glycol (proposed)) von der Firma ISP vertrieben.

Als dritten wesentlichen Bestandteil enthalten die erfindungsgemäßen Mittel ein Copolymer C, basierend auf den Monomeren C1 und C2. Für die technische Wirkung der erfindungsgemäßen Mittel haben sich Mittel als vorteilhaft erwiesen, bei denen das Copolymer C zu mindestens 70 Gew.-%, vorzugsweise zu mindestens 80 Gew.-%, bevorzugt zu mindestens 90 Gew.-% und insbesondere zu mindestens 95 Gew.-% aus den Monomeren C1 und C2 besteht. Weitere bevorzugte Copolymere C bestehen vollständig aus den Monomeren C1 und C2.

Das erfindungsgemäße Mittel enthält mindestens ein Copolymer C, das gebildet ist aus
- mindestens zwei Monomeren C1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
- als Monomer C2 Methacryloylethylaminoxid, insbesondere Methacryloylethyl-N,N-dimethylaminoxid (in Formel C2-I: R¹ = CH₃, n = 2, R² und R³ = CH₃).

Diese Copolymere sind bekannt und beispielsweise unter der Bezeichnung Diaformer Z-632 von der Firma Clariant erhältlich, wobei der Einsatz von Diaformer Z-632 besonders bevorzugt ist.

In einer bevorzugten Ausführungsform enthält das erfindungsgemäße Mittel mindestens ein Copolymer C, das gebildet ist aus
- mindestens drei Monomeren C1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, das zweite Monomer ausgewählt ist aus Acrylsäurelaurylester und Methacrylsäurelaurylester, und das dritte Monomer ausgewählt ist aus Acrylsäurestearylester und Methacrylsäurestearylester, und
- als Monomer C2 Methacryloylethylaminoxid, insbesondere Methacryloylethyl-N,N-dimethylaminoxid (in Formel C2-I: R¹ = CH₃, n = 2, R² und R³ = CH₃).

Entsprechende Copolymere sind ebenfalls bekannt und beispielsweise unter den Bezeichnungen Diaformer Z-611, Diaformer Z-612, Diaformer Z-613, Diaformer Z-631, Diaformer Z-633, Diaformer Z-651, Diaformer Z-711N, Diaformer Z-712N und Diaformer Z-731N von der Firma Clariant erhältlich, wobei der Einsatz von Diaformer Z-712N und Diaformer Z-651 bevorzugt ist.

Selbstverständlich ist es auch möglich, dass die erfindungsgemäßen Mittel eine Mischung aus mindestens zwei der Copolymere C enthalten, die gemäß der drei soeben beschriebenen bevorzugten Ausführungsformen eingesetzt werden. Der Gewichtsanteil des Copolymers C am Gesamtgewicht erfindungsgemäßer kosmetischer Mittels beträgt 0,1 bis 8,0 Gew.-%, vorzugsweise 0,2 bis 6,0 Gew.-% und insbesondere 0,4 bis 4,0 Gew.-%.

Die erfindungsgemäßen Mittel zeichnen sich von kosmetischen Mitteln mit alternativen Copoylmeren C neben den oben genannten Vorteilen insbesondere auch durch einen verbesserten Haltegrad aus. Als für die kosmetischen Eigenschaften der erfindungsgemäßen Mittel besonders vorteilhaft hat sich ein Verhältnis der gemeinsamen Gewichtsanteile der Copolymere A und B zum Gewichtsanteil des Polymer C in dem kosmetischen Mittel zwischen 8:1 und 1:8, vorzugsweise zwischen 6:1 und 1:6 und insbesondere zwischen 4:1 und 1:4
erwiesen.

Das Copolymere A, B und C werden in dem kosmetischen Mitteln vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten daher 2-Amino-2-methylpropanol. Das 2-Amino-2-methylpropanol wird in den erfindungsgemäßen Mitteln vorzugsweise in einer Menge eingesetzt, welche die zur Neutralisation der Copolymere A, B und C benötigte Menge nicht überschreitet. Vorzugsweise beträgt die in den erfindungsgemäßen Mitteln eingesetzte Mengen an 2-Amino-2-methylpropanol 80 bis 100%, besonders bevorzugt 90 bis 100% und insbesondere 95 bis 100% der zur vollständigen Neutralisation der Copolymere A, B und C benötigten Menge. In einer bevorzugten Ausführungsform beträgt der Gewichtsanteil des 2-Amino-2-methylpropanols am Gesamtgewicht des kosmetischen Mittels 0,1 bis 4,0 Gew.-%, bevorzugt 0,2 bis 3,0 Gew.% und insbesondere 0,5 bis 2,0 Gew.-%.

Neben den zuvor beschriebenen Copolymeren und Trägersubstanzen können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Als bevorzugten Bestandteil enthalten die erfindungsgemäßen kosmetischen Mittel mindestens eine quartäre Ammoniumverbindung. Als quartäre Ammoniumverbindung können monomere oder polymere Wirkstoffe eingesetzt werden.

Aus der Vielzahl an möglichen monomeren quartären Ammoniumverbindungen haben sich die Verbindungen aus den Gruppen:
- Trimethylalkylammoniumhalogenide;
- der Esterquats
- der quartären Imidazoline
als besonders wirkungsvoll erwiesen.

Zur Gruppe der Trimethylalkylammoniumhalogenide zählen insbesondere die Verbindungen der Formel (Tkat1-1).

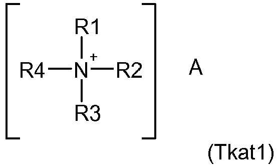

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.
Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat. Bevorzugte kosmetische Mittel enthalten eine monomere quartäre Ammoniumverbindung aus der Gruppe der Trimethylalkylammoniumhalogenide.

Weitere erfindungsgemäß besonders bevorzugte quartäre Ammoniumverbindungen sind die kationischen Betainestern der Formel (Tkat1-2.1).

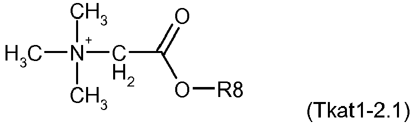

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im Folgenden dargestellte Formel (Tkat2) zeigt die Struktur dieser Verbindungen.

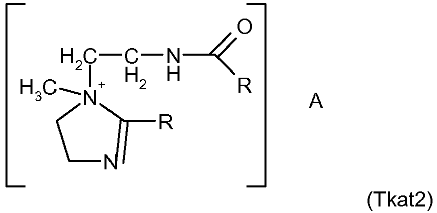

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCI - Bezeichnungen Quaternium-27, Quaternium-72, Quaternium-83, Quaternium-87 und Quaternium-91 erhältlich. Höchst bevorzugt ist erfindungsgemäß Quaternium-91.

In Bezug auf die kosmetische Wirkung haben sich kosmetische Mittel als vorteilhaft erwiesen, bei denen der Gewichtsanteil der monomeren quartären Ammoniumverbindung am Gesamtgewicht des Mittels 0,05 bis 3,0 Gew.-%, vorzugsweise 0,1 bis 2,0 Gew.-% und insbesondere 0,2 bis 1,0 Gew.-% beträgt.

Als geeignete Hilfs- und Zusatzstoffe sind insbesondere zusätzliche Pflegestoffe zu nennen. Als Pflegestoff kann beispielsweise ein Silikonöl und/oder ein Silikongum eingesetzt werden.

Erfindungsgemäß geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen.

Esteröle, das heißt Ester von C₆ - C₃₀ - Fettsäuren mit C₂ - C₃₀ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

In Bezug auf die kosmetische Wirkung haben sich kosmetische Mittel als vorteilhaft erwiesen, bei denen der Gewichtsanteil des Ölkörpers am Gesamtgewicht des Mittels 0,01 bis 5,0 Gew.-%, vorzugsweise 0,02 bis 4,0 Gew.-% und insbesondere 0,05 bis 2,0 Gew.-% beträgt.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer A | 0,05 bis 10 | 0,1 bis 7,0 | 0,2 bis 5,0 | 0,4 | 1,7 |
| Copolymer B | 0,05 bis 10 | 0,1 bis 7,0 | 0,2 bis 5,0 | 0,8 | 3,4 |
| Copolymer C | 0,1 bis 8,0 | 0,2 bis 6,0 | 0,4 bis 4,0 | 0,8 | 2,7 |
| Wasser, Misc | add 100 | add 100 | add 100 | add 100 | add 100 |

| | Formel 6 | Formel 7 | Formel 8 | Formel 9 | Formel 10 |
|---|---|---|---|---|---|
| Copolymer A ¹⁾ | 0,05 bis 10 | 0,1 bis 7,0 | 0,2 bis 5,0 | 0,4 | 2,7 |
| Copolymer B | 0,05 bis 10 | 0,1 bis 7,0 | 0,2 bis 5,0 | 0,8 | 0,5 |
| Copolymer C | 0,1 bis 8,0 | 0,2 bis 6,0 | 0,4 bis 4,0 | 0,8 | 2,7 |
| Wasser, Misc | add 100 | add 100 | add 100 | add 100 | add 100 |

| | Formel 11 | Formel 12 | Formel 13 | Formel 14 | Formel 15 |
|---|---|---|---|---|---|
| Copolymer A | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C | 0,1 bis 8,0 | 0,2 bis 6,0 | 0,4 bis 4,0 | 0,8 | 2,7 |
| Wasser, Misc | add 100 | add 100 | add 100 | add 100 | add 100 |

| | Formel 16 | Formel 17 | Formel 18 | Formel 19 | Formel 20 |
|---|---|---|---|---|---|
| Copolymer A | 0,05 bis 10 | 0,1 bis 7,0 | 0,2 bis 5,0 | 0,4 | 2,7 |
| Copolymer B | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Wasser, Misc | add 100 | add 100 | add 100 | add 100 | add 100 |

| | Formel 21 | Formel 22 | Formel 23 | Formel 24 | Formel 25 |
|---|---|---|---|---|---|
| Copolymer A ¹⁾ | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Wasser, Misc | add 100 | add 100 | add 100 | add 100 | add 100 |

| | Formel 26 | Formel 27 | Formel 28 | Formel 29 | Formel 30 |
|---|---|---|---|---|---|
| Copolymer A | 0,05 bis 10 | 0,1 bis 7,0 | 0,2 bis 5,0 | 0,4 | 1,7 |
| Copolymer B | 0,05 bis 10 | 0,1 bis 7,0 | 0,2 bis 5,0 | 0,8 | 3,4 |
| Copolymer C | 0,1 bis 8,0 | 0,2 bis 6,0 | 0,4 bis 4,0 | 0,8 | 2,7 |
| Alkanolamin ⁴⁾ | 80 bis 100 | 90 bis 100 | 95 bis 100 | 98 | 98 |
| Wasser, Misc | add 100 | add 100 | add 100 | add 100 | add 100 |

| | Formel 31 | Formel 32 | Formel 33 | Formel 34 | Formel 35 |
|---|---|---|---|---|---|
| Copolymer A ¹⁾ | 0,05 bis 10 | 0,1 bis 7,0 | 0,2 bis 5,0 | 0,4 | 2,7 |
| Copolymer B | 0,05 bis 10 | 0,1 bis 7,0 | 0,2 bis 5,0 | 0,8 | 0,5 |
| Copolymer C | 0,1 bis 8,0 | 0,2 bis 6,0 | 0,4 bis 4,0 | 0,8 | 2,7 |
| Alkanolamin ⁴⁾ | 80 bis 100 | 90 bis 100 | 95 bis 100 | 98 | 98 |
| Wasser, Misc | add 100 | add 100 | add 100 | add 100 | add 100 |

| | Formel 36 | Formel 37 | Formel 38 | Formel 39 | Formel 40 |
|---|---|---|---|---|---|
| Copolymer A | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C | 0,1 bis 8,0 | 0,2 bis 6,0 | 0,4 bis 4,0 | 0,8 | 2,7 |
| Alkanolamin ⁴⁾ | 80 bis 100 | 90 bis 100 | 95 bis 100 | 98 | 98 |
| Wasser, Misc | add 100 | add 100 | add 100 | add 100 | add 100 |

| | Formel 41 | Formel 42 | Formel 43 | Formel 44 | Formel 45 |
|---|---|---|---|---|---|
| Copolymer A | 0,05 bis 10 | 0,1 bis 7,0 | 0,2 bis 5,0 | 0,4 | 2,7 |
| Copolymer B | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Alkanolamin ⁴⁾ | 80 bis 100 | 90 bis 100 | 95 bis 100 | 98 | 98 |
| Wasser, Misc | add 100 | add 100 | add 100 | add 100 | add 100 |

| | Formel 46 | Formel 47 | Formel 48 | Formel 49 | Formel 50 |
|---|---|---|---|---|---|
| Copolymer A ¹⁾ | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Alkanolamin ⁴⁾ | 80 bis 100 | 90 bis 100 | 95 bis 100 | 98 | 98 |
| Wasser, Misc | add 100 | add 100 | add 100 | add 100 | add 100 |

| | Formel 51 | Formel 52 | Formel 53 | Formel 54 | Formel 55 |
|---|---|---|---|---|---|
| Copolymer A ¹⁾ | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Alkyltrimethylammoniumchlorid | 0,05 bis 3,0 | 0,1 bis 2,0 | 0,2 bis 1,0 | 0,5 | 0,3 |
| Wasser, Misc | add 100 | add 100 | add 100 | add 100 | add 100 |

| | Formel 56 | Formel 57 | Formel 58 | Formel 59 | Formel 50 |
|---|---|---|---|---|---|
| Copolymer A ¹⁾ | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Ölkörper | 0,01 bis 5,0 | 0,02 bis 4,0 | 0,05 bis 2,0 | 0,1 | 0,1 |
| Wasser, Misc | add 100 | add 100 | add 100 | add 100 | add 100 |

| | Formel 61 | Formel 62 | Formel 63 | Formel 64 | Formel 65 |
|---|---|---|---|---|---|
| Copolymer A ¹⁾ | 0,05 bis 10 | 0,1 bis 7,0 | 0,2 bis 5,0 | 0,4 | 2,7 |
| Copolymer B ²⁾ | 0,05 bis 10 | 0,1 bis 7,0 | 0,2 bis 5,0 | 0,8 | 0,5 |
| Copolymer C ³⁾ | 0,1 bis 8,0 | 0,2 bis 6,0 | 0,4 bis 4,0 | 0,8 | 2,7 |
| Wasser | 40 bis 99 | 50 bis 98 | 60 bis 95 | 97 | 92 |
| Misc | add 100 | add 100 | add 100 | add 100 | add 100 |

1) Copolymer A, welches zu mindestens 90 Gew.-% und insbesondere zu mindestens 95 Gew.-% aus den Struktureinheiten (A1), (A2), (A3) und (A4) besteht, wobei
   - R1 in Formel (A1) für einen Rest -CH(CH₃)CH₂-(OCH(CH₃)CH₂)ₓ(O[CH₂]₂)_{y}OCH₃ steht, in dem x und y unabhängig voneinander einen Wert zwischen 1 und 100 aufweisen;
   - R2 in Formel (A2) für einen Rest -(CH₂)₃-N(CH₃)₂ steht;
   - ein Rest R3 für -CH₂CH₃ und ein Rest R3 in Formel (A3) für H steht.
2) Copolymer B, welches vollständig aus der Struktureinheit (B1) besteht, in der der Rest R4 für -CH₃ steht.
3) Copolymer C, welches zu mindestens 90 Gew.-% und insbesondere zu mindestens 95 Gew.-%gebildet ist aus
   - mindestens zwei Monomeren C1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
   - als Monomer C2 Methacryloylethyl-N,N-dimethylaminoxid
4) % der zur vollständigen Neutralisation des Copolymers A benötigten Menge

Die Formulierung der erfindungsgemäßen Mittel kann in allen für kosmetische Mittel üblichen Formen erfolgen, beispielsweise in Form von Lösungen, die als Haarwasser, Pump- oder Aerosolspray auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind. Bevorzugt liegen die erfindungsgemäßen Mittel in flüssiger Form, beispielsweise in Form eines Sprays oder Aerosolsprays vor. In einer alternativen Ausführungsform können dieser Mittel jedoch auch in Gel- oder in Cremeform vorliegen, wobei transparente Gele besonders bevorzugt sind.

Wie weiter oben ausgeführt, enthalten die bevorzugten Aerosolsprays neben weiteren Aktiv- und Hilfsstoffen ein Treibmittel. Erfindungsgemäß geeignete Treibmittel (Treibgase) sind Propan, n-Butan, iso-Butan, Dimethylether (DME), Stickstoff, Luft, Lachgas, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Dimethylether, Propan, n-Butan, iso-Butan sowie Mischungen dieser Treibgase. Ganz besonders bevorzugt ist der Einsatz von Propan/Butan Gemischen oder Isobutan. Kosmetische Mittel, die das Treibmittel bezogen auf ihr Gesamtgewicht in einer Menge von 2,0 - 20 Gew.%, bevorzugt 4,0 - 15 Gew.% und besonders bevorzugt 5,0 - 10 Gew.-% enthalten, sind erfindungsgemäß bevorzugt.

Die Zusammensetzung einiger bevorzugter Treibmittel-haltiger kosmetischer Mittel kann der folgenden Tabellen entnommen werden. In dieser Tabelle verweist die linke Spalte ("Formel x") auf jeweils eine der in den weiter oben offenbarten Tabellen angeführten beispielhaften kosmetischen Zusammensetzungen. Die weiteren Spalten zwei bis sieben ("Treibmittel") geben jeweils die der entsprechenden kosmetischen Zusammensetzung zugesetzte Menge an Treibmittel an. Diese Angaben in "Gew.-%" beziehen sich auf das Gesamtgewicht der kosmetischen Zusammensetzung der jeweiligen "Formel x" ohne Treibmittel.

Eine kosmetische Zubereitung gemäß Zeile 12, Spalte 5 der nachfolgenden Tabelle, umfasst mit anderen Worten eine 20:1 Mischung des Treibmittel-freien kosmetischen Mittels gemäß Formel 11 mit einem Propan/Butan Gemisch.

| | Treibmittel [Gew.-%] | | | | | |
|---|---|---|---|---|---|---|
| Formel 1 | 2,0 bis 10 | 5,0 | 2,0 bis 10 P/B* | 5,0 P/B | 2,0 bis 10 iB* | 5,0 iB |
| Formel 2 | 2,0 bis 10 | 5,0 | 2,0 bis 10 P/B* | 5,0 P/B | 2,0 bis 10 iB* | 5,0 iB |
| Formel 3 | 2,0 bis 10 | 5,0 | 2,0 bis 10 P/B* | 5,0 P/B | 2,0 bis 10 iB* | 5,0 iB |
| Formel 4 | 2,0 bis 10 | 5,0 | 2,0 bis 10 P/B* | 5,0 P/B | 2,0 bis 10 iB* | 5,0 iB |
| Formel 5 | 2,0 bis 10 | 5,0 | 2,0 bis 10 P/B* | 5,0 P/B | 2,0 bis 10 iB* | 5,0 iB |
| Formel 6 | 2,0 bis 10 | 5,0 | 2,0 bis 10 P/B* | 5,0 P/B | 2,0 bis 10 iB* | 5,0 iB |
| Formel 7 | 2,0 bis 10 | 5,0 | 2,0 bis 10 P/B* | 5,0 P/B | 2,0 bis 10 iB* | 5,0 iB |
| Formel 8 | 2,0 bis 10 | 5,0 | 2,0 bis 10 P/B* | 5,0 P/B | 2,0 bis 10 iB* | 5,0 iB |
| Formel 9 | 2,0 bis 10 | 5,0 | 2,0 bis 10 P/B* | 5,0 P/B | 2,0 bis 10 iB* | 5,0 iB |
| Formel 10 | 2,0 bis 10 | 5,0 | 2,0 bis 10 P/B* | 5,0 P/B | 2,0 bis 10 iB* | 5,0 iB |
| Formel 11 | 2,0 bis 10 | 5,0 | 2,0 bis 10 P/B* | 5,0 P/B | 2,0 bis 10 iB* | 5,0 iB |
| Formel 12 | 2,0 bis 10 | 5,0 | 2,0 bis 10 P/B* | 5,0 P/B | 2,0 bis 10 iB* | 5,0 iB |
| Formel 13 | 2,0 bis 10 | 5,0 | 2,0 bis 10 P/B* | 5,0 P/B | 2,0 bis 10 iB* | 5,0 iB |
| Formel 14 | 2,0 bis 10 | 5,0 | 2,0 bis 10 P/B* | 5,0 P/B | 2,0 bis 10 iB* | 5,0 iB |
| Formel 15 | 2,0 bis 10 | 5,0 | 2,0 bis 10 P/B* | 5,0 P/B | 2,0 bis 10 iB* | 5,0 iB |
| Formel 16 | 2,0 bis 10 | 5,0 | 2,0 bis 10 P/B* | 5,0 P/B | 2,0 bis 10 iB* | 5,0 iB |
| Formel 17 | 2,0 bis 10 | 5,0 | 2,0 bis 10 P/B* | 5,0 P/B | 2,0 bis 10 iB* | 5,0 iB |
| Formel 18 | 2,0 bis 10 | 5,0 | 2,0 bis 10 P/B* | 5,0 P/B | 2,0 bis 10 iB* | 5,0 iB |
| Formel 19 | 2,0 bis 10 | 5,0 | 2,0 bis 10 P/B* | 5,0 P/B | 2,0 bis 10 iB* | 5,0 iB |
| Formel 20 | 2,0 bis 10 | 5,0 | 2,0 bis 10 P/B* | 5,0 P/B | 2,0 bis 10 iB* | 5,0 iB |
| Formel 21 | 2,0 bis 10 | 5,0 | 2,0 bis 10 P/B* | 5,0 P/B | 2,0 bis 10 iB* | 5,0 iB |
| Formel 22 | 2,0 bis 10 | 5,0 | 2,0 bis 10 P/B* | 5,0 P/B | 2,0 bis 10 iB* | 5,0 iB |
| Formel 23 | 2,0 bis 10 | 5,0 | 2,0 bis 10 P/B* | 5,0 P/B | 2,0 bis 10 iB* | 5,0 iB |
| Formel 24 | 2,0 bis 10 | 5,0 | 2,0 bis 10 P/B* | 5,0 P/B | 2,0 bis 10 iB* | 5,0 iB |
| Formel 25 | 2,0 bis 10 | 5,0 | 2,0 bis 10 P/B* | 5,0 P/B | 2,0 bis 10 iB* | 5,0 iB |
| Formel 26 | 2,0 bis 10 | 5,0 | 2,0 bis 10 P/B* | 5,0 P/B | 2,0 bis 10 iB* | 5,0 iB |
| Formel 27 | 2,0 bis 10 | 5,0 | 2,0 bis 10 P/B* | 5,0 P/B | 2,0 bis 10 iB* | 5,0 iB |
| Formel 28 | 2,0 bis 10 | 5,0 | 2,0 bis 10 P/B* | 5,0 P/B | 2,0 bis 10 iB* | 5,0 iB |
| Formel 29 | 2,0 bis 10 | 5,0 | 2,0 bis 10 P/B* | 5,0 P/B | 2,0 bis 10 iB* | 5,0 iB |
| Formel 30 | 2,0 bis 10 | 5,0 | 2,0 bis 10 P/B* | 5,0 P/B | 2,0 bis 10 iB* | 5,0 iB |
| Formel 31 | 2,0 bis 10 | 5,0 | 2,0 bis 10 P/B* | 5,0 P/B | 2,0 bis 10 iB* | 5,0 iB |
| Formel 32 | 2,0 bis 10 | 5,0 | 2,0 bis 10 P/B* | 5,0 P/B | 2,0 bis 10 iB* | 5,0 iB |
| Formel 33 | 2,0 bis 10 | 5,0 | 2,0 bis 10 P/B* | 5,0 P/B | 2,0 bis 10 iB* | 5,0 iB |
| Formel 34 | 2,0 bis 10 | 5,0 | 2,0 bis 10 P/B* | 5,0 P/B | 2,0 bis 10 iB* | 5,0 iB |
| Formel 35 | 2,0 bis 10 | 5,0 | 2,0 bis 10 P/B* | 5,0 P/B | 2,0 bis 10 iB* | 5,0 iB |
| Formel 36 | 2,0 bis 10 | 5,0 | 2,0 bis 10 P/B* | 5,0 P/B | 2,0 bis 10 iB* | 5,0 iB |
| Formel 37 | 2,0 bis 10 | 5,0 | 2,0 bis 10 P/B* | 5,0 P/B | 2,0 bis 10 iB* | 5,0 iB |
| Formel 38 | 2,0 bis 10 | 5,0 | 2,0 bis 10 P/B* | 5,0 P/B | 2,0 bis 10 iB* | 5,0 iB |
| Formel 39 | 2,0 bis 10 | 5,0 | 2,0 bis 10 P/B* | 5,0 P/B | 2,0 bis 10 iB* | 5,0 iB |
| Formel 40 | 2,0 bis 10 | 5,0 | 2,0 bis 10 P/B* | 5,0 P/B | 2,0 bis 10 iB* | 5,0 iB |
| Formel 41 | 2,0 bis 10 | 5,0 | 2,0 bis 10 P/B* | 5,0 P/B | 2,0 bis 10 iB* | 5,0 iB |
| Formel 42 | 2,0 bis 10 | 5,0 | 2,0 bis 10 P/B* | 5,0 P/B | 2,0 bis 10 iB* | 5,0 iB |
| Formel 43 | 2,0 bis 10 | 5,0 | 2,0 bis 10 P/B* | 5,0 P/B | 2,0 bis 10 iB* | 5,0 iB |
| Formel 44 | 2,0 bis 10 | 5,0 | 2,0 bis 10 P/B* | 5,0 P/B | 2,0 bis 10 iB* | 5,0 iB |
| Formel 45 | 2,0 bis 10 | 5,0 | 2,0 bis 10 P/B* | 5,0 P/B | 2,0 bis 10 iB* | 5,0 iB |
| Formel 46 | 2,0 bis 10 | 5,0 | 2,0 bis 10 P/B* | 5,0 P/B | 2,0 bis 10 iB* | 5,0 iB |
| Formel 47 | 2,0 bis 10 | 5,0 | 2,0 bis 10 P/B* | 5,0 P/B | 2,0 bis 10 iB* | 5,0 iB |
| Formel 48 | 2,0 bis 10 | 5,0 | 2,0 bis 10 P/B* | 5,0 P/B | 2,0 bis 10 iB* | 5,0 iB |
| Formel 49 | 2,0 bis 10 | 5,0 | 2,0 bis 10 P/B* | 5,0 P/B | 2,0 bis 10 iB* | 5,0 iB |
| Formel 50 | 2,0 bis 10 | 5,0 | 2,0 bis 10 P/B* | 5,0 P/B | 2,0 bis 10 iB* | 5,0 iB |
| Formel 51 | 2,0 bis 10 | 5,0 | 2,0 bis 10 P/B* | 5,0 P/B | 2,0 bis 10 iB* | 5,0 iB |
| Formel 52 | 2,0 bis 10 | 5,0 | 2,0 bis 10 P/B* | 5,0 P/B | 2,0 bis 10 iB* | 5,0 iB |
| Formel 53 | 2,0 bis 10 | 5,0 | 2,0 bis 10 P/B* | 5,0 P/B | 2,0 bis 10 iB* | 5,0 iB |
| Formel 54 | 2,0 bis 10 | 5,0 | 2,0 bis 10 P/B* | 5,0 P/B | 2,0 bis 10 iB* | 5,0 iB |
| Formel 55 | 2,0 bis 10 | 5,0 | 2,0 bis 10 P/B* | 5,0 P/B | 2,0 bis 10 iB* | 5,0 iB |
| Formel 56 | 2,0 bis 10 | 5,0 | 2,0 bis 10 P/B* | 5,0 P/B | 2,0 bis 10 iB* | 5,0 iB |
| Formel 57 | 2,0 bis 10 | 5,0 | 2,0 bis 10 P/B* | 5,0 P/B | 2,0 bis 10 iB* | 5,0 iB |
| Formel 58 | 2,0 bis 10 | 5,0 | 2,0 bis 10 P/B* | 5,0 P/B | 2,0 bis 10 iB* | 5,0 iB |
| Formel 59 | 2,0 bis 10 | 5,0 | 2,0 bis 10 P/B* | 5,0 P/B | 2,0 bis 10 iB* | 5,0 iB |
| Formel 60 | 2,0 bis 10 | 5,0 | 2,0 bis 10 P/B* | 5,0 P/B | 2,0 bis 10 iB* | 5,0 iB |
| Formel 61 | 2,0 bis 10 | 5,0 | 2,0 bis 10 P/B* | 5,0 P/B | 2,0 bis 10 iB* | 5,0 iB |
| Formel 62 | 2,0 bis 10 | 5,0 | 2,0 bis 10 P/B* | 5,0 P/B | 2,0 bis 10 iB* | 5,0 iB |
| Formel 63 | 2,0 bis 10 | 5,0 | 2,0 bis 10 P/B* | 5,0 P/B | 2,0 bis 10 iB* | 5,0 iB |
| Formel 64 | 2,0 bis 10 | 5,0 | 2,0 bis 10 P/B* | 5,0 P/B | 2,0 bis 10 iB* | 5,0 iB |
| Formel 65 | 2,0 bis 10 | 5,0 | 2,0 bis 10 P/B* | 5,0 P/B | 2,0 bis 10 iB* | 5,0 iB |

| | | | | | | |
|---|---|---|---|---|---|---|
| * "P/B" entspricht einem Propan/Butan Gemisch ** "iB" entspricht Isobutan | | | | | | |

Wie eingangs ausgeführt, weisen die erfindungsgemäßen Mittel vorteilhafte haarfixierende Eigenschaften auf. Ein Verfahren zur temporären Verformung keratinischer Fasern, bei welchem eine erfindungsgemäße Zusammensetzung auf die keratinische Faser aufgebracht wird, ist daher ein weiterer Gegenstand der vorliegenden Anmeldung. Die Verwendung eines erfindungsgemäßen kosmetischen Mittels zur temporären Verformung keratinischer Fasern ist ein weiterer Gegenstand der vorliegenden Anmeldung. Wie eingangs ausgeführt, zeichnen sich die erfindungsgemäßen Mittel insbesondere durch einen verbesserten Halt bei der temporären Verformung keratinischer Fasern aus. Ein zusätzlicher Gegenstand der vorliegenden Anmeldung ist daher die Verwendung eines erfindungsgemäßen kosmetischen Mittels zur Verbesserung des Halts bei der temporären Verformung keratinischer Fasern.

## Patentansprüche

1. Kosmetisches Mittel, enthaltend in einem kosmetisch akzeptablen Träger
a) mindestens ein Copolymer A mit mindestens einer Struktureinheit (A1), mindestens
einer Struktureinheit (A2), mindestens eine Struktureinheit (A3) und mindestens einer Struktureinheit (A4), 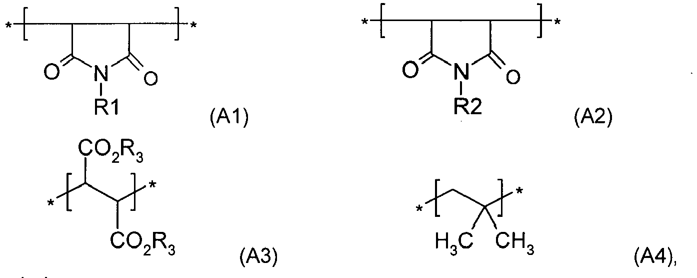 wobei
- R1 für einen gegebenenfalls heterofunktionaliserten Alkylrest steht;
- R2 für einen von R1 verschiedenen, gegebenenfalls heterofunktionalisierten Alkylrest steht;
- R3 voneinander unabhängig für einen von R1 und R2 verschiedenen gegebenenfalls heterofunktionalisierten Alkylrest steht, wobei der Gewichtsanteil des Copolymers A am Gesamtgewicht des Mittels 0,05 bis 10 Gew.-% beträgt,
b) mindestens ein von Copolymer A verschiedenes Copolymer B, welches vollständig aus einer Struktureinheit (B1) 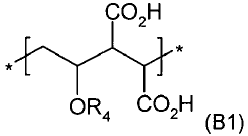 besteht, wobei R4 für einen gegebenenfalls heterofunktionaliserten Alkylrest steht und der Gewichtsanteil des Copolymers B am Gesamtgewicht des Mittels 0,05 bis 10 Gew.-%, beträgt; und
c) mindestens ein von Copolymer A und Copolymer B verschiedenes Copolymer C, gebildet aus
- mindestens zwei Monomeren C1, wobei das erste Monomer ausgewählt ist aus Acrylsäure, Methacrylsäure, Acrylsäuremethylester, Methacrylsäuremethylester, Acrylsäureethylester, Methacrylsäureethylester, Acrylsäurepropylester, Methacrylsäurepropylester, Acrylsäureisopropylester und Methacrylsäureisopropylester, und das zweite Monomer ausgewählt ist aus Acrylsäurestearylester und Methacrylsäurestearylester, und
- Methacryloylethylaminoxid als Monomer C2,
wobei der Gewichtsanteil des Copolymers C am Gesamtgewicht des Mittels 0,1 bis 8,0 beträgt.

2. Kosmetisches Mittel nach Anspruch 1, **dadurch gekennzeichnet, dass** der
- R1 in Formel (A1) für einen Rest -CH(CH₃)CH₂-(OCH(CH₃)CH₂)ₓ(O[CH₂]₂)_{y}OCH₃ steht, in dem x und y unabhängig voneinander einen Wert zwischen 1 und 100 aufweisen;
- R2 in Formel (A2) für einen Rest -(CH₂)₃-N(CH₃)₂ steht;
- ein Rest R3 für -CH₂CH₃ und ein Rest R3 in Formel (A3) für H steht

3. Kosmetisches Mittel nach einem der vorherigen Ansprüche, **dadurch gekennzeichnet, dass** der Gewichtsanteil des Copolymers A am Gesamtgewicht des Mittels 0,1 bis 7,0 Gew.-% und insbesondere 0,2 bis 5,0 Gew.-% beträgt.

4. Kosmetisches Mittel nach einem der vorherigen Ansprüche, **dadurch gekennzeichnet, dass** der Rest R4 in Formel (B1) für -CH₃ steht.

5. Kosmetisches Mittel nach einem der vorherigen Ansprüche, **dadurch gekennzeichnet, dass** der Gewichtsanteil des Copolymers B am Gesamtgewicht des Mittels 0,1 bis 7,0 Gew.-% und insbesondere 0,2 bis 5,0 Gew.-% beträgt.

6. Kosmetisches Mittel nach einem der vorherigen Ansprüche, **dadurch gekennzeichnet, dass** das kosmetische Mittel weiterhin mindestens ein 1,2 Diol, vorzugsweise 1,2 Octandiol enthält.

7. Kosmetisches Mittel nach einem der vorherigen Ansprüche, **dadurch gekennzeichnet, dass** der Gewichtsanteil des Copolymers C am Gesamtgewicht des Mittels 0,2 bis 6,0 Gew.-% und insbesondere 0,4 bis 4,0 Gew.-% beträgt.

8. Verwendung eines kosmetischen Mittels nach einem der vorherigen Ansprüche, zur temporären Verformung keratinischer Fasern.

9. Verfahren zur temporären Verformung keratinischer Fasern, bei welchem eine Zusammensetzung nach einem der Ansprüche 1 bis 7 auf die keratinische Faser aufgebracht wird.

## Claims

1. A cosmetic agent containing, in a cosmetically acceptable carrier,
a) at least one copolymer A having at least one structural unit (A1), at least one structural unit (A2), at least one structural unit (A3) and at least one structural unit (A4), 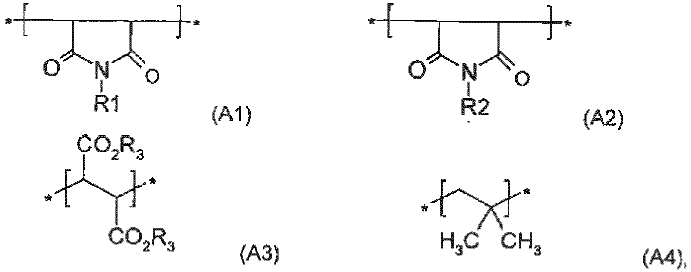 where
- R1 represents an optionally heterofunctionalized alkyl group;
- R2 represents an optionally heterofunctionalized alkyl group that is different from R1;
- R3 represents, independently, an optionally heterofunctionalized alkyl group that is different from R1 and R2, wherein the proportion by weight of copolymer A with respect to the total weight of the agent is from 0.05 to 10% wt.%,
b) at least one copolymer B which is different from copolymer A and consists entirely of one structural unit (B1) 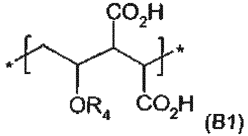 where R4 represents an optionally heterofunctionalized alkyl group and the proportion by weight of copolymer B with respect to the total weight of the agent is from 0.05 to 10 wt.%; and
c) at least one copolymer C which is different from copolymer A and copolymer B and is formed by
- at least two monomers C1, wherein the first monomer is selected from acrylic acid, methacrylic acid, acrylic acid methyl ester, methacrylic acid methyl ester, acrylic acid ethyl ester, methacrylic acid ethyl ester, acrylic acid propyl ester, methacrylic acid propyl ester, acrylic acid isopropyl ester and methacrylic acid isopropyl ester, and the second monomer is selected from acrylic acid stearyl ester and methacrylic acid stearyl ester, and
- methacryloylethylamine oxide as a monomer C2, wherein the proportion by weight of copolymer C with respect to the total weight of the agent is from 0.1 to 8.0.

2. The cosmetic agent according to claim 1, **characterized in that**
- R1 in formula (A1) represents a group -CH(CH₃)CH₂-(OCH(CH₃)CH₂)ₓ(O[CH₂]₂)_{y}OCH₃, in which x and y, independently of one another, have a value between 1 and 100;
- R2 in formula (A2) represents a group -(CH₂)₃-N(CH₃)₂;
- a group R3 represents -CH₂CH₃ and a group R3 in formula (A3) represents H.

3. The cosmetic agent according to one of the preceding claims, **characterized in that** the proportion by weight of copolymer A with respect to the total weight of the agent is from 0.1 to 7.0 wt.% and in particular from 0.2 to 5.0 wt.%.

4. The cosmetic agent according to one of the preceding claims, **characterized in that** the group R4 in formula (B1) represents -CH₃.

5. The cosmetic agent according to one of the preceding claims, **characterized in that** the proportion by weight of copolymer B with respect to the total weight of the agent is from 0.1 to 7.0 wt.% and in particular from 0.2 to 5.0 wt.%.

6. The cosmetic agent according to one of the preceding claims, **characterized in that** the cosmetic agent also contains at least one 1,2-diol, preferably 1,2-octanediol.

7. The cosmetic agent according to one of the preceding claims, **characterized in that** the proportion by weight of copolymer C with respect to the total weight of the agent is from 0.2 to 6.0 wt.% and in particular from 0.4 to 4.0 wt.%.

8. The use of a cosmetic agent according to one of the preceding claims for temporarily shaping keratin fibers.

9. A method for temporarily shaping keratin fibers, in which a composition according to one of claims 1 to 7 is applied to the keratin fibers.

## Revendications

1. Agent cosmétique contenant, dans un support cosmétiquement acceptable,
a) au moins un copolymère A comportant au moins une unité structurelle (A1), au moins une unité structurelle (A2), au moins une unité structurelle (A3) et au moins une unité structurelle (A4), 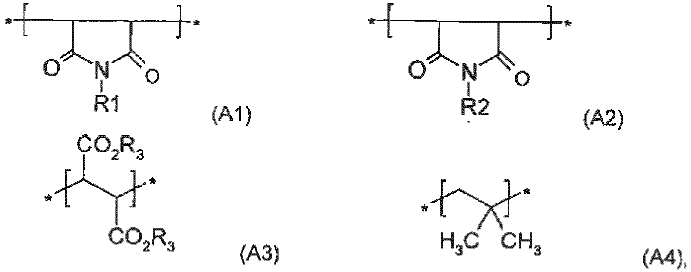 dans lesquelles
- R1 est un radical alkyle éventuellement hétérofonctionnalisé ;
- R2 est un radical alkyle éventuellement hétérofonctionnalisé différent de R1 ;
- R3 est, indépendamment l'un de l'autre, un radical alkyle éventuellement hétérofonctionnalisé différent de R1 et R2, la proportion en poids du copolymère A par rapport au poids total de l'agent étant de 0,05 à 10 % en poids,
b) au moins un copolymère B différent du copolymère A, qui consiste entièrement en une unité structurelle (B1) 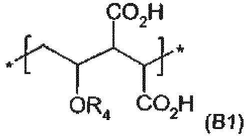 dans laquelle R4 est un radical alkyle éventuellement hétérofonctionnalisé et la proportion en poids du copolymère B par rapport au poids total de l'agent est de 0,05 à 10 % en poids ; et
c) au moins un copolymère C différent du copolymère A et du copolymère B, formé
- d'au moins deux monomères C1, le premier monomère étant choisi parmi l'acide acrylique, l'acide méthacrylique, le méthylester d'acide acrylique, le méthylester d'acide méthacrylique, l'éthylester d'acide acrylique, l'éthylester d'acide méthacrylique, le propylester d'acide acrylique, le propylester d'acide méthacrylique, l'isopropylester d'acide acrylique et l'isopropylester d'acide méthacrylique, et le second monomère étant choisi parmi le stéarylester d'acide acrylique et le stéarylester d'acide méthacrylique, et
- d'oxyde de méthacryloyléthylamine en tant que monomère C2, la proportion en poids du copolymère C dans le poids total de l'agent étant de 0,1 à 8,0.

2. Agent cosmétique selon la revendication 1, **caractérisé en ce que**
- R1 représente, dans la formule (A1), un radical -CH(CH₃)CH₂-(OCH(CH₃)CH₂)ₓ(O[CH₂]₂)_{y}OCH₃, dans lequel x et y présentent, indépendamment l'un de l'autre, une valeur entre 1 et 100 ;
- R2 représente, dans la formule (A2), un radical -(CH₂)₃-N(CH₃)₂ ;
- un radical R3 représente -CH₂CH₃ et un radical R3 représente H dans la formule (A3).

3. Agent cosmétique selon l'une quelconque des revendications précédentes, **caractérisé en ce que** la proportion en poids du copolymère A dans le poids total de l'agent est de 0,1 à 7,0 % en poids et en particulier de 0,2 à 5,0 % en poids.

4. Agent cosmétique selon l'une quelconque des revendications précédentes, **caractérisé en ce que** le radical R4 représente -CH₃ dans la formule (B1).

5. Agent cosmétique selon l'une quelconque des revendications précédentes, **caractérisé en ce que** la proportion en poids du copolymère B dans le poids total de l'agent est de 0,1 à 7,0 % en poids et en particulier de 0,2 à 5,0 % en poids.

6. Agent cosmétique selon l'une quelconque des revendications précédentes, **caractérisé en ce que** l'agent cosmétique contient en outre au moins un 1,2-diol, de préférence le 1,2-octanediol.

7. Agent cosmétique selon l'une quelconque des revendications précédentes, **caractérisé en ce que** la proportion en poids du copolymère C dans le poids total de l'agent est de 0,2 à 6,0 % en poids et en particulier de 0,4 à 4,0 % en poids.

8. Utilisation d'un agent cosmétique selon l'une quelconque des revendications précédentes pour la mise en forme temporaire de fibres kératiniques.

9. Procédé pour la mise en forme permanente de fibres kératiniques, dans lequel une composition selon l'une quelconque des revendications 1 à 7 est appliquée sur les fibres kératiniques.
